Europäisches Patentamt

(19) European Patent Office

Office européen des brevets

(11) Publication number: **0 178 400**
A1

## (12) EUROPEAN PATENT APPLICATION

(21) Application number: 85109472.2

(22) Date of filing: 27.07.85

(51) Int. Cl.⁴: **C 07 D 311/72**
C 07 B 37/04
//A61K31/355

(30) Priority: 25.09.84 US 654343
19.11.84 US 672774

(43) Date of publication of application:
23.04.86 Bulletin 86/17

(84) Designated Contracting States:
AT BE CH DE FR GB IT LI LU NL SE

(71) Applicant: HENKEL CORPORATION
7900 West 78th Street
Minneapolis Minnesota 55435(US)

(72) Inventor: Willging, Stephen M.
2007 Franklin Avenue S.E.
Minneapolis Minnesota 55084(US)

(74) Representative: von Kreisler, Alek, Dipl.-Chem. et al,
Deichmannhaus am Hauptbahnhof
D-5000 Köln 1(DE)

(54) Methylation of non-alpha-tocopherol compounds.

(57) This invention relates to a method for the methylation of non-alpha-tocopherol compounds thereby producing the alpha-tocopherols having greater Vitamin E activity than the starting compounds. A feed material containing non-alpha-tocopherol compounds is contacted with formaldehyde to cause a hydroxymethylation and which is reduced, producing an alpha-tocopherol compound. Thereafter, the alpha-tocopherol compound produced is recovered.

EP 0 178 400 A1

## METHYLATION OF NON-ALPHA-TOCOPHEROL COMPOUNDS

### BACKGROUND OF THE INVENTION

#### 1. Field of the Invention

This invention relates to the enhancement of Vitamin E activity of tocopherol homologues having lower Vitamin E activity than d-alpha-tocopherols due to the absence of methyl groups on the aromatic tocopherol ring. The tocopherol homologues which can be methylated to enhance Vitamin E activity have the beta-, gamma- and delta-tocopherol structure which have hydrogen in the 5 and/or 7 position of the aromatic ring instead of a methyl group. Such tocopherol homologues exhibiting Vitamin E activity which can be enhanced include the toco-mono-, di-, and tri-enols which are different only in having an unsaturated isoprenoid $C_{16}$ side chain.

Thus, the term "non-alpha-tocopherol homologues" used throughout refers to tocol compounds which have less than the three (5, 7, and 8) methyl groups common to the 5,7,8-trimethyl tocol and which instead exhibit one or more aromatic hydrogens. The term "tocol" is used herein to mean 2-methyl-2-(4',8',12',-trimethyl-tridecyl)chroman-6-ol. Other compounds included in the term "tocopherol" are the previously indicated toco-enols such as toco-mono, di or tri-enol, which have some Vitamin E activity and which exhibit the beta-, gamma- or delta-tocol structure of the 2-methyl-2(4',8',12'-trimethyltridecyl)chroman-6-ol, except for having some degree of unsaturation in the isoprenoid $C_{16}$-side chain. Such beta, gamma, and delta compounds, whether they are saturated or unsaturated, can be methylated in accordance with the instant invention to the alpha-tocopherol structure, thereby increasing their Vitamin E activity.

The term Vitamin E was originally used to designate the active component of certain vegetable oils. Vitamin E activity means the physiological activity of a group of nutrient materials originally isolated from various natural sources. The materials having Vitamin E activity all belong to a distinct series of compounds which are all derivatives of chroman-6-ol. These compounds are all tocol derivatives having an isoprenoid $C_{16}$-side chain. The compounds of primary importance for Vitamin E activity are the more highly methylated tocol homologues, especially 5,7,8-trimethyl tocol. The nomenclature currently used for the most highly methylated tocopherol having methyl groups at the 5, 7, and 8 positions of the aromatic ring is alpha-tocopherol. Beta-tocopherol has methyl groups at the 5 and 8 positions, gamma at the 7 and 8 positions, and delta at the 8 position, having two aromatic hydrocarbons.

The physiological activity of this group of compounds is measured by the ability to maintain fertility in rats. The Vitamin E active compounds have a variety of beneficial effects in humans with the completely methylated alpha-tocopherol homologue having the highest potency as measured by its effectiveness in maintaining rat fertility. For this reason, the term Vitamin E is frequently used interchangeably with d-alpha-tocopherol, the naturally occurring tocopherol of highest Vitamin E activity.

The alpha-tocopherol is produced commercially for use as a feed supplement for domestic animals, as a source of Vitamin E activity and as a nutrient supplement for humans. The alpha-tocopherol is also used in food technology as an anti-oxidant to retard the development of rancidity in fatty materials.

Tocopherol homologues are found widely distributed in normal foods, occurring in the highest concentration in the cereal grain oils, principally in corn and wheat oils, but also in barley and rye. They are also found in vegetable oils such as safflower, soybean, peanut, cottonseed, linseed, sunflower, rapeseed, and palm; and in other vegetable sources such as palm leaves, lettuce, alfalfa, rubber latex, and a variety of other plant materials. The proportion of the most active form, the d-alpha tocopherol varies widely among the different sources. Two of the highest sources of d-alpha tocopherol are safflower oil and sunflower oil, although the most commonly available source is soybean oil which has a considerably lower percent of d-alpha-tocopherol with significantly higher percentages of the gamma and delta homologues. In addition to being a source for the saturated tocopherols, palm oil, oats, rye and barley also contain the unsaturated homologues of tocotrienol, herein included in the term "tocopherol homologues".

The naturally occurring tocopherols are generally isolated from natural products such as vegetable oil sources by various combinations of such procedures as esterification, saponification, hydrogenation, extraction, distillation, ion exchange, absorption chromatography, precipitation of sterols, crystallization and many others. The tocopherol concentrate isolated will vary depending upon the particular vegetable source and separating techniques used.

## 2. Background of the Related Art

Various prior art alkylation methods are known. These include methods as described, for example, in U.S. Patent 2,486,539 and U.S. Patent 2,486,542. Both of these methods first chloromethylate the 5, and/or 7 positions using formaldehyde and hydrogen chloride or hydrochloric acid. These chloromethyl groups are then converted into methyl groups by a reduction procedure. U.S. Patent 2,486,542 uses a stannous chloride and hydrochloric acid reduction procedure. U.S. Patent 2,486,539 teaches catalytic hydrogenation with a hydro-

-3-

~onation catalyst such as nickel or paladium under hydrogen pressure or, alternatively the Clemmensen procedure using zinc dust and hydrochloric acid.

Another chloromethylation procedure is described in U.S. Patent 2,843,604 which provides for two successive chloromethylations along with reduction by a catalytic hydrogenation or by the addition of metallic zinc or stannous chloride to the acidic reaction mixture.

Several other commonly known procedures used to upgrade Vitamin E activity in non-alpha-tocopherols involve the hydroxymethylation of the non-alpha-tocopherols followed by a hydrogenation process. One such method is taught in U.S. Patent No. 3,819,657 to General Mills Chemicals, Inc., which teaches the use of formaldehyde with orthophosphoric acid. Hydrogenation is accomplished through the use of hydrogen pressure over a hydrogenation catalyst such as palladium, preferably on carbon.

Another similar process is found described in Japanese early Disclosure No. 81-68677 (Patent No. 56-68677). A one-step conversion of non-alpha-tocopherol homologues to alpha-tocopherol is taught in this patent by first hydroxymethylating the non-alpha-tocopherols using an organic acid and formaldehyde. Hydrogenation finishes the one-step process through the presence of hydrogen with palladium, nickel or platinum to ensure the hydrogenation of the hydroxymethylated intermediate completing the production of alpha- tocopherol. This disclosure indicates that the hydrogenation catalyst palladium on carbon is preferred.

Another hydroxymethylation-hydrogenation process is described in U.S. Patent No. 4,239,691. This process is catalyzed by an insoluble acidic ion exchange resin to produce the hydroxymethylated intermediate which is then hydrogenated with a hydrogenation catalyst, preferably palladium on carbon. A minimum temperature of 175°C with high pressure is used.

It is an object of the instant invention to provide a process for the methylation of the non-alpha-tocopherol homologues (beta, gamma and delta) by hydroxymethylation and reduction. It is a further object of the instant invention to provide a process which achieves methylation of non-alpha-tocopherol homologues having the tocol structure without using a strong acid such as HCl. It is therefore a further object of the instant invention to achieve methylation under less corrosive conditions. Other objects will become apparent as this description proceeds.

## BRIEF DESCRIPTION

The instant invention provides a method for increasing the Vitamin E activity of non-alpha-tocopherol homologues by methylating non-alpha-tocopherol homologues. In one aspect of the invention, this is achieved by reacting the non-alpha tocopherols with formaldehydes under reducing conditions or atmospheres through a process comprising contacting said non-alpha-tocopherol homologues with formaldehyde, a carboxylic acid, and a metallic reduction reagent for a sufficient length of time to produce an alpha-tocopherol homologue. Thereafter the alpha-tocopherol homologue produced is collected from the reaction mixture. In a second aspect of the invention this is achieved by contacting the non-alpha-tocopherol with formaldehyde, hydrogen gas and an acid-compatible hydrogenation metal on an acidic polymeric ion exchange support at a minimum temperature of about 40°C, whereby methylation occurs producing alpha-tocopherol; and then recovering the alpha-tocopherol.

## ASPECT 1 OF THE INVENTION

This process also produces water and a carboxylate salt formed with the metallic reduction agent, which will be present in the product mixture with the alpha-tocopherol. The desired alpha-tocopherol product can be collected or separated from the product mixture

containing these by-products by conventional methods such as distillation and extraction.

The metallic reduction reagent is a metal which causes the reduction from the hydroxymethylated intermediate to the more highly methylated tocopherol. This metal, while causing the reduction, is in the reaction, converted to a carboxylate salt or ion. This salt must be soluble in the mixture to keep remaining metal free for further reaction.

This process can be conducted using a batch or continuous apparatus, either one of which could conduct a one or two step methylation followed by the recovery of the alpha-tocopherol. The one step methylation procedure would combine, in any convenient manner, non-alpha-tocopherol homologues with sufficient amounts of formaldehyde, a carboxylic acid and a metallic reduction reagent. This permits hydroxymethylation to occur readily with a simultaneous reduction of the hydroxymethylated intermediate. In fact, hydroxymethylation and reduction occur simultaneously in the reaction mixture. After a sufficient length of time, the alpha-tocopherol product can be collected.

If this process is conducted using a two step methylation, the non-alpha-tocopherol homologues are contacted first with both formaldehyde and at least a catalytic amount of a carboxylic acid. The reaction occurring in this first step produces the hydroxymethylated intermediate which is then contacted with a metallic reduction reagent and more carboxylic acid if the amount of carboxylic acid present in the first step was less then two equivalents per equivalent of aromatic hydrogens in the non-alpha-tocopherol homologue for a sufficient length of time to reduce the hydroxy methylated intermediate to the alpha-tocopherol product.

Advantageously, in this process the hydrogen gas producing side reaction occurring between the metallic reduction reagent and the carboxylic acid occurs less readily than it would if a stronger acid such as HCl were present. If the process is conducted in two separate steps, however, this side reaction is even more limited since it occurs only in the second step where the metallic reduction reagent is combined with the hydroxymethylated intermediate in the presence of carboxylic acid.

## ASPECT 2 OF THE INVENTION

Formaldehyde can be provided by the direct addition of formaldehyde or by using a material which releases formaldehyde in the reaction vessel.

The metal supported on the acid polymeric ion exchange support must be acid compatible. Such metals do not degrade or react with the strongly acidic ion exchange support on which they are placed. Both platinum and palladium are two hydrogenation metals which are sufficiently resistant to strong acidic ion exchange supports thus permitting their use.

A single atmosphere of hydrogen gas is a suitable pressure for the instant invention. Advantageously and preferably, however, higher hydrogen pressures can be used.

The temperature used is a minimum of 40°C. The maximum temperature suitable is determined by the highest temperature at which the particular polymeric support can be used without a substantial degradation. It should be remembered, however, that one large advantage to the instant catalyst is the capability of methylating non-alpha tocopherols at lower temperature. If desired, however, acidic polymeric supports can be used which are stable up to and over 200°C. Sulfonated polytetrafluoroethylene, for example, is suitable support, and is stable at such temperatures.

This process achieves the methylation of non-alpha-tocopherols to alpha-tocopherols in one step by using the solid catalyst described. Since a solid catalyst is used, advantageously the instant process

-7-

can be employed in a fixed bed system in a batch or continuous manner.

Due to the proximity of the hydrogenating metal to the acidic ion exchange support which is responsible for hydroxymethylating the non-alpha-tocopherols, both · hydroxymethylation and hydrogenation will occur in the first process step. This catalyst thus achieves a rapid and complete conversion of non-alpha-tocopherol homologues to alpha-tocopherol.

DETAILED DESCRIPTION OF THE INVENTION

Any material, at least a portion of which is non alpha tocopherol homologues having the tocol structure can be used as feed material. While ideally the feed material should be highly concentrated in the non-alpha-tocopherol homologues, it is possible to use feed material wherein the non-alpha-tocopherol concentration is as low as 3%. A preferred feed material has a minimum of 25% by weight non-alpha-tocopherol homologue is acceptable and a more preferred feed material has a minimum of about 45% by weight non-alpha-tocopherol homologues. One more preferred feed material which can be obtained from processing vegetable oils such as soybean oil is deodorizer distillate, which usually has a minimum of about 50% by weight mixed tocopherols.

ASPECT 1 OF THE INVENTION

Both a wide variety of non-reacting liquid materials and the reacting carboxylic acids can be used as a solvent for the process of the instant invention. The non-reacting solvent material can be polar or nonpolar and thus can be miscible or immiscible with the reactant mixture. The solvent can be added in any convenient manner to the feed material.

Acceptable solvents for use with the instant invention include materials such as kerosene, benzene, dixoane, toluene, aliphatic hydrocarbon solvents and ethers. Preferably the carboxylic acid used in accordance with the instant invention is added in a suffi-

-8-

ciently large amount to act also as a solvent. Thus, preferred solvents also include the carboxylic acid reagents preferably lower alkanoic and lower alkanedioic acids. Most preferred acidic solvents are those such as acetic, propionic, and butyric acid. Any non-acidic solvent material used should be non-reactive to both alpha and non-alpha-tocopherol.

Suitably the instant process can be used with ambient pressures. If, however, a more volatile carboxylic acid is selected, such as formic acid, it might be convenient to apply pressure in order to maintain liquid conditions. Pressure would particularly be applied in the case of more volatile acids used at higher temperatures. Individual circumstances, however, would largely control what pressure is used. Suitably the pressure is sufficient to maintain liquid conditions while contacting the non-alpha-tocopherols with formaldehyde, or the hydroxymethylated intermediate with the carboxylic acid and the metallic reduction reagent.

While non-alpha-tocopherol homologues can be methylated using the process of the instant invention at ambient temperatures, the reaction proceeds slowly at ambient temperatures so that it is more acceptable to use higher temperatures. Acceptably the temperatures used should be in the range of from about 75 to 170°C. A preferred temperature range is from about 90 to 150°C.

Hydroxymethylation of non-alpha-tocopherol homologues occurs using formaldehyde. The instant invention thus requires formaldehyde to contact the non-alpha-tocopherols. This formaldehyde can be provided by the direct addition of formaldehyde, in any convenient manner, such as by the addition to the feed material of an aqueous formaldehyde solution. Formaldehyde can, however, be provided by a source of formaldehyde, such as paraformaldehyde, trioxane, or can be provided

-9-

by a formaldehyde-releasing polymer added to the feed material in any convenient manner.

Acceptably, one equivalent of formaldehyde should be present for each equivalent of the methylatable aromatic hydrogen (per equivalent of the methylatable sites) in the non-alpha-tocopherol homologues. The maximum amount of formaldehyde used is a question of practicality for the individual system used. Thus, there is no real maximum formaldehyde limit. An acceptable concentration range for formaldehyde or a source of formaldehyde is from about one equivalent of formaldehyde to about ten equivalents of formaldehyde per equivalent methylation sites of the non-alpha-tocopherol homologues. A more preferred range for formaldehyde concentration, particularly if using a formaldehyde source, is from about 1.2 equivalents of formaldehyde to about 6.0 equivalents of formaldehyde per equivalent of non-alpha-tocopherol compounds.

In accordance with the instant process, the formaldehyde is added to the feed material in any convenient manner before, during or after the addition of the addition of carboxylic acid. If the process is being conducted in a two step manner, the formaldehyde is used in the first step along with at least a catalytic amount of the carboxylic acid. In this first step, the formaldehyde and the non-alpha-tocopherol homologues react to form the hydroxymethylated intermediate. This reaction can continue in the second step of a two step methylation, if additional non-alpha-tocopherol is added or if the non-alpha-tocopherol present in the first step was not completely hydroxymethylated by the time the metallic reduction reagent was added.

The instant process calls for the use of a carboxylic acid. Whether this process is conducted batchwise or continuously, either one of which could have a one or two step methylation, any carboxylic acid is

-10-

appropriate. Suitable acids can be selected from the group consisting of formic acid, acetic acid, propionic acid, and butyric acid. The most preferred acids are acetic acid, propionic acid, and butyric acid. Permissibly, water can be added up to about 10% by weight, in a range of from about 1% to about 10% by weight.

If this process is being conducted using a two step methylation, it is permissible to use the acid in only a catalytic amount in only the first step. In this case, a catalytic amount of the acid would be added to the feed material in any convenient manner along with a sufficient amount of formaldehyde or a formaldehyde source. After a sufficient amount of time, a hydroxymethylated intermediate is produced in this first step, at which point more carboxylic and the metallic reduction reagent is added in any convenient manner. This starts the second step of the methylation process, which produces the alpha-tocopherol.

When using this two step process in which the carboxylic acid was used in only a catalytic amount in the first step, the carboxylic acid is added at the beginning of the second step in an amount necessary to insure that a minimum of two equivalents of carboxylic acid are present per equivalent of methylatable aromatic hydrogen or the non-alpha-tocopherol compounds to be methylated. There is no actual limit on the amount of acid permitted. Preferably the acid is added in an amount of from about 5 to about 40 equivalents per equivalent of methylation sites (methylatable aromatic hydrogens) of the non-alpha homologues to be methylated. The metallic reduction reagent added at the beginning of the second step of this two step process is acceptably added in an amount of from about one to about 6 equivalents per equivalent of methylation sites in the non-alpha tocopherol homologues to be methylated.

-11-

**0178400**

A more preferred embodiment of the two step methylation is to add the carboxylic acid in a minimum amount of two equivalents of carboxylic acid per equivalent of methylation sites in the non-alpha-tocopherol at the beginning of or during the first step in any convenient manner. This eliminates the need for further addition of carboxylic acid. In this case, the required metallic reduction reagent is added, beginning the second step after a sufficient amount of hydroxymethylated intermediate is produced. During this second step, both reduction and hydroxymethylation occur.

Whether using a one or two step methylation process, a preferred concentration range for the carboxylic acid is from about 5 to about 40 equivalents of carboxylic acid per equivalent of methylation sites in the non-alpha-tocopherol compounds. Most preferably, however, the carboxylic acid is added in a solvent amount along with the formaldehyde or a formaldehyde source for either a one or two step process.

Metallic reduction reagents which could be used are the metals magnesium, calcium, tin, nickel and zinc. Of these, the most preferred is zinc. Acceptably, the metal reduction agent is added in a sufficient amount either at the beginning of a one step process or at the beginning of the second step in a two step process where hydroxymethylation has been allowed to continue for a sufficient length of time to produce some concentration of the hydroxymethylated intermediate. Although the addition of the metallic reduction catalyst can be made in any convenient manner, it is preferred to add it in a mixture with the carboxylic acid when a two step methylation is used.

A salt is formed from the metal reduction reagent. This salt must be capable of dissolving in the carboxylic acid so that the metal surface remains clear, thus allowing further reaction of the remaining metal.

-12-

The quantity of the metallic reduction reagent in either a one or two step process is a minimum of one equivalent of metal catalyst per equivalent of the methylation sites (aromatic hydrogens) non-alpha-tocopherol homologues, and a preferred quantity of metallic reduction reagent to be added is from about 1 to about 3 equivalents of metal reduction reagent per equivalent of non-alpha-tocopherol homologues.

The metallic reduction reagent is added along with a sufficient quantity of carboxylic acid and a formaldehyde source if a one step methylation is used. If a two step methylation process is being conducted, the metallic reduction reagent is not added until after a sufficient amount of time has allowed the non-alpha-tocopherol homologues to become hydroxymethylated. Suitably, both steps are conducted at the same temperatures. Thus a temperature in the range of from about 75° to about 170° Centigrade (C) is suitable for reduction of the hydroxymethylated intermediate.

When the metallic reduction reagent is present in the reaction mixture along with the carboxylic acid, however, a side reaction occurs producing hydrogen gas. However, this reaction occurs to a lesser extent when using a carboxylic acid than it would if a strong acid such as HCl was used.

A preferred embodiment of the instant invention calls for the continued addition of the metallic reduction agent to the reaction mixture as the hydroxymethylated intermediate is produced. In this case, a minimum of two equivalents of carboxylic acid and one equivalent of formaldehyde is present per equivalent of the methylation sites (aromatic hydrogens) in the non-alpha-tocopherol homologues so that further addition of acid and formaldehyde is not necessary.

Since both reduction and hydroxymethylation can occur in the second step of a two step process, it is permissible to continually add feed material, formaldehyde, acid and metallic reduction reagent to the reaction mixture.

The length of time required to produce alpha-tocopherol homologues by the methylation of non-alpha-tocopherol homologues leaving a tocol structure by the process of the instant invention will depend on individual circumstances such as the particular temperature used, the particular quantities of reagents used, and the character of the feed material. The time necessary to methylate the non-alpha-tocopherol homologues therefore varies considerably. When a one step methylation is conducted, the time necessay to produce a separatable alpha-tocopherol homologue is from about 1 minute to about 20 hours. Likewise, in a two step methylation procedure, the amount of time necessay to produce the hydroxymethylated tocopherol intermediate in the first step will depend on the specific characteristics of the individual system. Generally, however, the time will range from about 2 minutes to 10 hours. The second step of a two step methylation procedure generally begins with the addition of the metallic reduction reagent. This second step likewise will vary in length depending on the individual circumstances used. Generally, however, this second step will likewise vary in time from about 2 minutes to about 10 hours. During this second step, both hydroxymethylation and reduction of the hydroxymethylated intermediate occurs. At the conclusion of this second step in a two step methylation procedure, the alpha-tocopherol homologues produced are separated from the remaining mixture by any convenient method.

In a preferred embodiment of the instant invention, a salt of the metal reduction reagent can be added in a catalytic amount to aid in the hydroxymethylation by acting as a Lewis acid. Such a salt however is not required for reduction. This salt can be added either slightly before or along with the metallic reduction reagent or simultaneous with the carboxylic acid. This salt must also be soluble in the reaction medium. Suitably, this metal salt is added in an amount of up to about one half the number of equivalents of the metal reduction reagent used. A preferred concentration range for this salt is from about 0.1 to about 10 equivalents per equivalent of the metal reduction reagent. Representative but non-exhaustive examples of salts which can be used are chlorides, bromide, and iodide. The most preferred salt is zinc chloride.

After hydroxymethylation and subsequent reduction, the alpha tocopherol homologues are recovered by separating them from the remaining mixture. This separation can be achieved by any convenient method, such as distillation, extraction, ion exchange, absorption chromatography, crystallization, and esterification.

### ASPECT 2 OF THE INVENTION

The use of a solvent material is permissible with the process of the instant invention. Such solvent material must be non-reacting to the tocopherol and the formaldehyde and should be compatible with the solid catalyst of the instant invention. Preferably, the solvent should be polar so that it can absorb the water produced by the methylation of the non-alpha tocopherols. This will allow the water to be removed and separated from the catalyst which might otherwise be degraded by the absorption of water. The solvent used can be added in any convenient manner. It can be added separately, added with a feed material or with the formaldehyde or formaldehyde source. Representative but nonexhaustive examples of preferred solvents are: alcohols and ethers. In fact, for extended use of these solid catalysts in accordance with the instant invention where the temperatures are from about 40 to 100°C, such polar solvents should be used. This will discourage catalytic degradation due to the presence of water.

Alternatively, however the water forming during methylation can be driven off by maintaining the reaction temperatures in a higher range such as from about 100 to 145°C or, even in the slightly lower but preferred range of from about 100 to about 120°C.

Suitably, the instant process can be used with ambient pressures (one atmosphere of hydrogen). Higher pressures are, however, preferred. Especially if formaldehyde is used directly or if a more volatile solvent material is used. Additionally, higher presssures will tend to encourage a faster reaction. Acceptably the pressure can be maintained in the range of from about 1 atmosphere to about 70 atmospheres. A preferred pressure range is from about 1 atmosphere to about 25 atmospheres.

In order to hydroxymethylate the non-alpha-tocopherol homologues, the presence of formaldehyde is necessary since formaldehyde must contact the non-alpha-tocopherol homologues in the presence of the acidic ion exchange support. This formaldehyde can be provided by the addition of formaldehyde or a formaldehyde source in any convenient manner; such as, for example, the direct addition to the feed material of a formaldehyde solution, or a formaldehyde source in a solution. Representative but nonexhaustive examples of a source of formaldehyde are: formalin, paraformaldehyde, trioxane, or a formaldehyde releasing polymer.

Acceptably, a minimum of at least one equivalent of formaldehyde should be present for each equivalent of the non-alpha-tocopherol homologues (otherwise stated, at least 1 equivalent of formaldehyde per equivalent of the aromatic hydrogens on the non-alpha-tocopherol homologues). The maximum amount of formaldehyde used is a question of practicality for the individual system used. Thus there is no real maximum formaldehyde limit. An acceptable concentration range for formaldehyde or a source of formaldehyde is from about 1 equivalent of formaldehyde to about 18 equivalents of formaldehyde or a formaldehyde source per equivalent of the aromatic hydrogens on the non-alpha-tocopherol homologues. A preferred range for formaldehyde or a source of formaldehyde is from about 1.25 to about 12 equivalents per equivalent of aromatic hydrogens, and a more preferred range for formaldehyde concentration, particularly if using a formaldehyde source, is from about 1.25 to about 8 equivalents of the formaldehyde source per equivalent of the non-alpha-tocopherol homologues.

The solid catalyst used in the process of the instant invention comprises an acid compatible hydrogenation metal on an acidic ion exchange support. This support can be either a polymeric acidic organic ion exchange resin, or a polymeric acidic inorganic ion exchange support. Zeolites or clays are suitable polymeric acidic inorganic ion exchange supports.

A wide variety of polymeric organic acidic ion exchange resins are available for use with the process of the instant invention. Acceptable polymeric organic acidic ion exchange resins can be made from divinyl benzenes, phenols, aliphatic aldehydes, acrylates, styrene and ethylene and halogenated derivatives of these. Suitably, polymeric resins made from these materials are sulfonated, phosphorylated. Preferred polymeric organic acidic ion exchange resins for the instant invention are: the sulfonated polystyrene, polyethylene, and polyphenolic resins. Such resins are widely available on the commercial market under such trade names as, for example: AMBERLITE, produced by Rohm & Haas; AMBERLYST, also produced by Rohm and Haas; DOWEX , produced by Dow Chemical Company; NAFION , produced by Dupont; and DUOLITE , produced by Diamond Alkali Company. Further descriptions of suitable polymeric organic acidic ion exchange resins can be found in Ion Exchange, by Frederick Helfferich (publisher

-17-

McGraw-Hill Book Company, Inc., 1962; pp. 14-16 and 29-47).

The metal loaded onto the acidic ion exchange support must be compatible with (resistant to degredation by) the acid. Representative examples of such acid compatible metals are platinum and palladium. These metals are also typically known as hydrogenation catalysts, since they are used widely to catalyze hydrogenation reactions. Their function as part of the solid catalyst of the instant invention is to complete the conversion of the non-alpha-tocopherol homologues to alpha tocopherol by catalyzing a hydrogenolysis reaction between hydrogen and the hydroxymethylated intermediate.

The acid-compatible hydrogenation metal can be loaded onto the polymeric acidic ion exchange support by any method that is convenient. Supported metal catalysts are common and any method which achieves this loading can be used to make the solid catalysts applied in the instant invention.

One common method used for making supported metal catalysts which can be used to produce the solid catalyst of the instant invention begins with making a solution of a salt of the acid compatible hydrogenation metal (for example, an aqueous solution of a salt of platinum or palladium). The polymeric acidic ion exchange support is then placed in suspension in this solution. The hydrogenation metal is then reduced onto the support.

An alternate method, also suitable to produce the solid catalyst called for by the instant invention, is to expose the metal salt to the selected polymeric acidic ion exchange support and allow an ion exchange to take place. The metal is then reduced on the support. One way to accomplish this is by using heat reduction. This reduction completes the solid methylation catalyst to be used in the instant invention.

Due to the proximity of the acid-compatible hydrogenation catalyst metal to the polymeric acidic ion exchange support in the solid catalyst of the instant invention, moderate temperatures as low as 40°C can be used. The maximum temperature allowable in a particular application of the process of the instant invention depends upon the highest temperature stability of the particular polymeric acidic ion exchange support used.

The process of the instant invention is capable of completing the methylation of non-alpha-tocopherol homologues at relatively moderate, preferred temperatures of from about 40 to 145°C compared to other hydroxy methylation/hydrogenation processes, although higher temperatures can be used if desired. Acceptably, in accordance with the instant invention, the reaction temperatures can be maintained in the broad range of from about 40 to about 200°C.

The preferred lower temperatures would tend to prolong the life of the catalyst, thus a more acceptable temperature range is from about 40 to about 145°C. A more preferred temperature range for the process of the instant invention would maintain the temperatures at from about 60 to about 120°C.

The time necessary for such methylation of the non-alpha-tocopherols into alpha-tocopherols will depend upon the reaction conditions in each particular instance. Factors which will influence the length of time necessary for methylation to occur are such things as the amount of formaldehyde; the amount of non-alpha-tocopherol homologues, absence or presence and amount of a solvent; and the relative amount of the catalyst pressure used, and temperature. Acceptably the time used in the first step of the instant invention wherein the non-alpha-tocopherol homologues are methylated, can vary from about .5 to about 20 hours. A more preferred range is from about 1 to about 4 hours.

After methylation, the alpha-tocopherol is separated from the remaining mixture. Any process for the recovery or separation of a chemical compound from a mixture could be applied to separate and isolate the alpha-tocopherol product in this process. Acceptable methods which can be used to separate, or isolate, and thereby recover the alpha-tocopherol product are: distillation, adsorption, extraction, ion exchange, crystallization and esterification.

These and other readily attainable variations in the process of the instant invention will be more fully understood from the examples which follow. These examples are intended to clarify and demonstrate the instant invention and not to limit it. All parts and percentages are by weight unless otherwise specified.

## EXAMPLES

### EXAMPLE I

A suspension of 5 g. of zinc dust, 2 g. of zinc chloride and 40 mls. of propionic acid was stirred at reflux and 5 g. of paraformaldehyde was added. A solution of 20 g. of mixed tocopherol concentrate (63% tocopherols made up of about 6% by weight alpha, about 39% by weight beta-gamma, and about 18% by weight delta), 2 g. of squalane as an internal standard, and 40 mls. of propionic acid was made up and added to the zinc and propionic acid mixture over a one-half hour period. The mixture was refluxed for one hour. The mixture was then cooled to room temperature and 100 mls. of water was added. This mixture was then extracted with 100 mls. of hexane. The aqueous layer was separated and back extracted with an additional 100 mls. of hexane. The hexane layers were combined, washed with water and dried over anhydrous sodium sulfate. The solvent was removed under a 10 mm. vacuum to give 22 g. of a yellow oil which was analyzed by capillary GLC (using the 10% by weight squalane as an internal standard) to give a yield of 39% alpha tocopherol.

EXAMPLE II

A solution of 10 g. of paraformaldehyde and 200 mls. of acetic acid was combined at room temperature with 20 g. of a mixed tocopherol concentrate that was 56.9% by weight (by wt.) mixed tocopherols of a composition of about 6% by wt. alpha, about 35.9% by wt. beta-gamma, and about 14.9% by wt. delta, and 100 mls. of acetic acid. Fifty grams of zinc dust was then added, and this mixture was then heated to 75°C and held at that temperature for one hour while stirring vigorously. The temperature was then elevated to 100° and was maintained at that temperature for one hour. After cooling to room temperature, 100 mls. of water was added to the reaction mixture and this was then extracted with 100 mls. of hexane. The aqueous layer was then back-extracted with an additional 100 mls. of hexane. The hexane layers were combined and washed with water and dried over anhydrous sodium sulfate. The volatile solvent material was then removed to give a yellow-orange oil product. This product was then analyzed by capillary GC using 10% squalane by wt. as an internal standard and a 50% by wt. yield of alpha-tocopherol was indicated.

EXAMPLE III

A solution of 20 g. of a mixed tocopherol concentrate (63% by wt. tocopherols made up of 6% by wt. alpha, 39% by wt. beta-gamma, and about 18% by wt. delta), 2 g. of squalene as an internal standard, and 50 ml. of acetic acid, at room temperature, was added slowly over one-half hour to a suspension of 50 g. of zinc dust, 2.5 g. of zinc chloride, 10 mls. of water, and 50 mls. of acetic acid under reflux. The reaction was refluxed for an additional hour. The mixture was cooled and 100 ml. of hexane was added. The solution was decanted from the unused zinc. The unused zinc was then washed with 50 mls. of a 50/50 mixture of hexane and acetone. The organic layers were then combined, washed with water, and dried over $Na_2SO_4$. The solvent was removed with a rotary evaporator to give a product of 22 g. of an orange oil. The product was analyzed with a capillary GC to show a 79% yield of alpha-tocopherol.

EXAMPLE IV

A 5 g. quantity of trioxane was added to a refluxing suspension of 50 g. of zinc dust, 2.5 g. of zinc chloride, and 50 ml of acetic acid. A mixture of 20 g. of a tocopherol concentrate (63% by wt. mixed tocopherols made up of 6% by wt. alpha, 39% by wt. beta-gamma, and 18% by wt. delta), and squalene in 75 ml. of acetic acid was added to the refluxing suspension over a one-half hour period. A two phase reaction was noted. The reaction was refluxed for an additional hour. After cooling, 100 ml. of hexane was added. The solution was decanted from the unused zinc which was then washed several times with 50 ml. of a 50/50 mixture of hexane and acetone to recover remaining product. The organic layers were combined, washed with water, and dried over $Na_2SO_4$. The solvent was removed by rotary evaporation to give a product of 21 g. of an orange oil. A capillary G.C. analysis indicated an alpha-tocopherol yield of 86%.

EXAMPLE V

Part A - Preparation of the Catalyst

A solution was prepared of acetone and 3.5 g. of palladium acetate. The acidic ion exchange resin used was AMBERLYST 15 of Rohm and Haas which is commercially available. Fifteen grams of this acidic ion exchange resin, 80 to 100 mesh, was added to the solution of palladium acetate and acetone. The suspension was stirred until only a trace of color remained in the acetone solution indicating the adsorption of the acid-compatible hydrogenation metal. The acidic ion exchange resin was then removed from the acetone and was washed several times with quantities of acetone. The resin was then treated with formic acid for 3 hours to reduce the palladium. The resulting resin was black and was washed with methanol and was dried under vacuum to give 16.5 g. of a palladium on acidic ion exchange resin catalyst. This solid catalyst was then used to methylate tocopherols in accordance with the process described herein (see Part B)

Part B - Methylation of Tocopherols

The non alpha-tocopherol containing feed material used in methylation was a mixed tocopherol concentrate having a 79.2% total tocopherols which was 73.3% non alpha-tocopherols (the beta, gamma, and delta homologues.) Twenty grams of this concentrate was combined with 10 g. of trioxane (used as the formaldehyde source) and 200 ml. of diethyleneglycol dimethylether as the solvent; 5.0 g. of the palladium on acidic resin catalyst which was prepared in Part A was also added. This mixture was placed in a rocking hydrogenation apparatus and the system was pressurized to 70 psi with hydrogen gas and was shaken at 80°C for 4 hours. 100 mls. of hexane was used to extract the product and this hexane was washed 3 times with water. The hexane solution was then dried with anhydrous sodium sulfate, and the hexane was removed under vacuum to give 20.5 g. of a light orange oil. The analysis of this oil by capillary glc using squalene as an internal standard showed a 60% yield of alpha-tocopherol and a 6% yield of beta-tocopherol.

WHAT IS CLAIMED IS:

1.  In a process for producing alpha-tocopherol from
    non-alpha tocopherol wherein the non-alpha tocopherol is reacted with formaldehyde to provide a
    hydroxymethylated compound which is reduced the
    improvement comprising (a) reacting said formaldehyde with said non-alpha tocopherol under reducing
    conditions, thereby providing alpha-tocopherol and
    (b) recovering the alpha-tocopherol.

2.  A process as defined in claim 1 wherein the non-
    alpha-tocopherol is contacted with formaldehyde, a
    carboxylic acid, and a metallic reduction reagent
    whereby the alpha-tocopherol is produced.

3.  A process as described in claim 2 wherein the metallic reduction reagent is zinc metal.

4.  A process as described in claim 2 wherein the non-
    alpha-tocopherol is present in a feed material having a minimum of 25% by weight non-alpha-tocopherol
    and wherein a salt selected from the group consisting of zinc chloride is present in an amount of up
    to one-half the number of equivalents of zinc present.

5.  A process as described in claim 3 wherein the zinc
    and the formaldehyde are present in a minimum
    amount of about 1 equivalent per equivalent of the
    methylation sites in the non-alpha-tocopherol present.

6.  A process as described in claim 5 wherein step (a)
    is conducted at a temperature in the range from
    about 75°C to 170°C.

7. A process as described in claim 6 wherein the carboxylic acid is selected from the group consisting of formic acid, acetic acid, propionic acid, and butyric acid.

8. A process as described in claim 7 wherein the carboxylic acid is present in a minimum amount of two equivalents per equivalent of methylation sites in the non-alpha-tocopherol, and where the temperature is from about 90°C to about 150°C.

9. A process as described in claim 1 wherein the non-alpha-tocopherol is contacted with formaldehyde and hydrogen gas in the presence of an acid-compatible hydrogenation metal on a polymeric acidic ion exchange support at a minimum temperature of about 40°C, whereby, methylation occurs producing alpha-tocopherol.

10. A process as described in claim 9 wherein the acid-compatible hydrogenation metal is selected from the group consisting of platinum and palladium.

11. A process as described in claim 9 wherein the support is a polymeric organic acidic ion exchange resin.

12. A process as described in claim 9 wherein the temperature is maintained in the range of from about 40°C to about 145°C.

13. A process as described in claim 11 wherein the polymeric acidic organic ion exchange resin is made from a material selected from the group consisting of: divinyl benzenes, phenols, aliphatic aldehydes, acrylates, styrene, and ethylene.

14. A process as described in claim 12 wherein the hydrogen gas pressure is maintained in a range of from about 1 atmosphere to about 70 atmospheres.


15. A process as described in claim 12 wherein the formaldehyde is present at a concentration of from about one to about 18 equivalents per equivalent of the aromatic hydrogens of the non-alpha-tocopherol homologues, and the hydrogen pressure is maintained in a range of from about 1 to about 25 atmospheres.

**European Patent Office**

**EUROPEAN SEARCH REPORT**

## DOCUMENTS CONSIDERED TO BE RELEVANT

EP 85109472.2

| Category | Citation of document with indication, where appropriate, of relevant passages | Relevant to claim | CLASSIFICATION OF THE APPLICATION (Int. Cl.4) |
|---|---|---|---|
| D,A | US - A - 3 819 657 (W.S.BALDWIN et al.) <br> * Claims 1,4 * | 1,6 | C 07 D 311/72 <br> C 07 B 37/04 <br> //A 61 K 31/355 |
| A | US - A - 3 631 068 (D.R.NELAN et al.) <br> * Claims 1,2 * | 1 | |
| A | US - A - 2 992 235 (J. GREEN) <br> * Claim 1 * | 1 | |

TECHNICAL FIELDS SEARCHED (Int. Cl.4)

C 07 D 311/00

The present search report has been drawn up for all claims

| Place of search | Date of completion of the search | Examiner |
|---|---|---|
| VIENNA | 21-01-1986 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82